# EUROPEAN PATENT SPECIFICATION

(11) **EP 4 097 446 B1**
(45) Date of publication and mention of the grant of the patent: **24.12.2025**
(21) Application number: 21704028.6
(22) Date of filing: 20.01.2021
(51) Int. Cl.: G01N 15/0205, G01N 33/49, G01N 15/01

(54) **MEASURING APPARATUS AND MEASURING METHOD**
MESSVORRICHTUNG UND MESSVERFAHREN
APPAREIL DE MESURE ET PROCÉDÉ DE MESURE

(30) Priority: 30.01.2020 JP 2020014053
(43) Date of publication of application: 07.12.2022
(73) Proprietor: Nihon Kohden Corporation, Tokyo 161-8560 (JP)
(72) Inventor: HIGUCHI, Makoto, Tokorozawa-shi, Saitama 359-0037 (JP); GENDA, Hironaga, Tokorozawa-shi, Saitama 359-0037 (JP); HAMADA, Tatsuya, Tokorozawa-shi, Saitama 359-0037 (JP); ESAKI, Tatsuhiro, Tokorozawa-shi, Saitama 359-0037 (JP)
(74) Representative: Grünecker Patent- und Rechtsanwälte PartG mbB
(86) International application number: PCT/JP2021/001900
(87) International publication number: WO 2021/153385

(56) References cited:
- WO-A1-2019/202621
- JP-A- S5 883 233

## Description

### Technical Field

The present invention relates to an erythrocyte aggregation measuring apparatus and measuring method.

### Background

Among known erythrocyte aggregation (gathering) reaction measuring methods is a method of stopping a flow of blood instantaneously after giving a certain shear stress to it and measuring an optical density variation (called a syllectogram) that occurs at that time.

To measure a syllectogram accurately, it is necessary to stop a blood flow instantaneously after giving a predetermined shear stress to it. On the other hand, a tube pump as described in, for example, the following Patent document 1 can be used as a means for moving a blood sample stored in a storage unit to a detection unit which is a measuring unit. The tube pump sucks a blood sample by operating in such a manner that rollers press a tube when they rotate in the circumferential direction.

Patent document 2 discloses an apparatus and a method to determine an erythrocyte sedimentation rate and other connected parameters.

### Citation List

### Patent Literature

Patent document 1: JP-S-58-83233
Patent document 2: WO 2019/202621 A1

### Summary of Invention

### Technical Problem

However, it is difficult to perform a blood flow control such as an accurate control of a blood flowing position and speed and stopping of a blood flow because of viscoelasticity of the entire flow passage formed by the tube pump and a resin tube connected to it. More specifically, if air which is a compressive fluid exists between blood and the tube pump in sucking the blood by the tube pump, the pressure is attenuated as it is transmitted due to high viscoelasticity of the air, making it difficult to perform an accurate control of a blood flow that is required for a syllectogram measurement.

This problem is not limited to the case of using a tube pump and also occurs in a case of using a suction pump.

One method for reducing the attenuation of pressure when it is transmitted would be to fill up the flow passage with a rinse liquid which is a non-compressive fluid. However, if the rinse liquid and the blood come into contact with each other, concentration of the blood or its components may vary, possibly disabling an accurate syllectogram measurement.

The presently disclosed subject matter has been conceived to solve the above problem, and has as an object providing a measuring apparatus and a measuring method capable of controlling a blood flow accurately and preventing contact between a rinse liquid and the blood.

### Solution to Problem

The measuring apparatus according to the presently disclosed subject matter which attains the above object is a measuring apparatus for measuring aggregation of erythrocytes of a blood sample, as defined in claim 1.

The measuring method according to the presently disclosed subject matter which attains the above object is a measuring method of a measuring apparatus for measuring aggregation of erythrocytes of a blood sample, as defined in claim 11.

### Advantageous Effects of Invention

According to the above-described measuring apparatus and measuring method, entrance of an air layer into the flow passage after a rinse liquid can be detected by the sensor. Thus, an air layer of such an amount as not to obstruct a blood flow control can be formed between blood and a rinse liquid by sucking a blood sample after a small amount of air layer. As a result, a measuring apparatus and a measuring method capable of controlling a blood flow accurately and preventing contact between a rinse liquid and the blood can be provided.

### Brief Description of Drawings

[fig.1]FIG. 1 is a rough diagram illustrating a measuring apparatus according to an embodiment of the presently disclosed subject matter.
[fig.2]FIG. 2 is a rough diagram illustrating a tube pump of the measuring apparatus according to the embodiment.
[fig.3]FIG. 3 is a rough diagram illustrating a detection unit of the measuring apparatus according to the embodiment.
[fig.4]FIG. 4 is a graph of an example syllectogram.
[fig.5]FIG. 5 is a rough diagram illustrating a measuring apparatus according to Modification 1.
[fig.6]FIG. 6 is a rough diagram illustrating a measuring apparatus according to Modification 2.

### Description of Embodiment

An embodiment of the presently disclosed subject matter will be described with reference to the drawings. In the descriptions of the drawings, the same constituent elements will be given the same symbols and will not be described redundantly. Ratios between dimensions in the drawings may be exaggerated for convenience of description and hence be different from actual ratios.

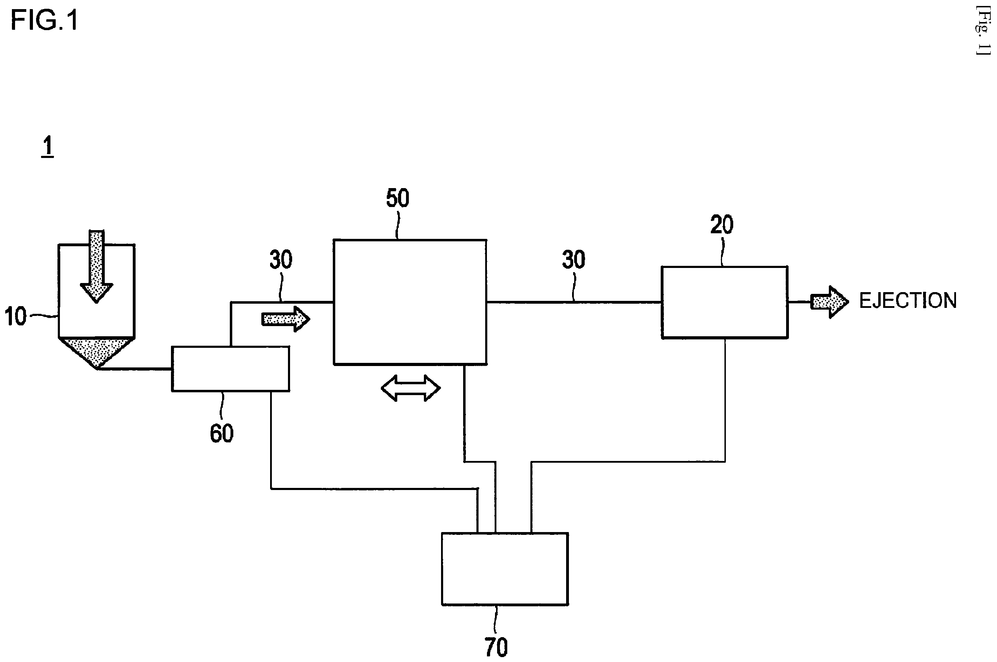

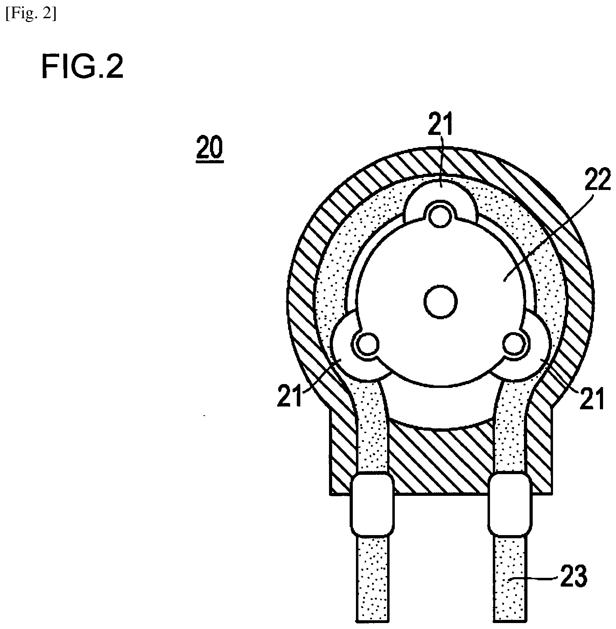

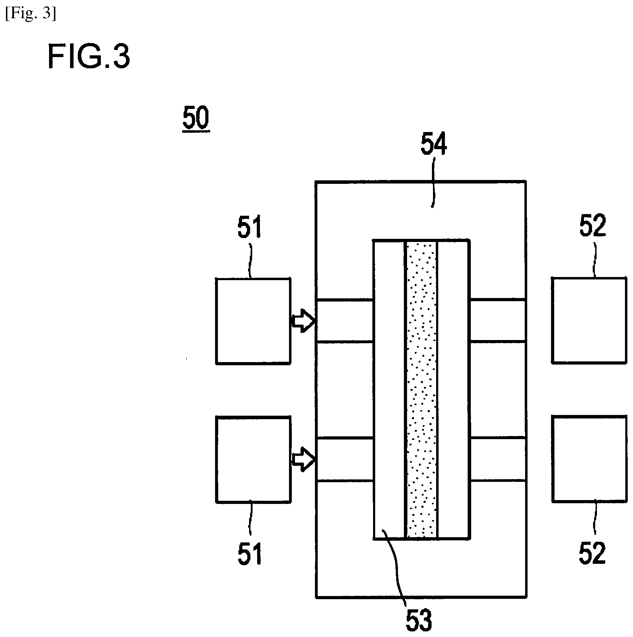

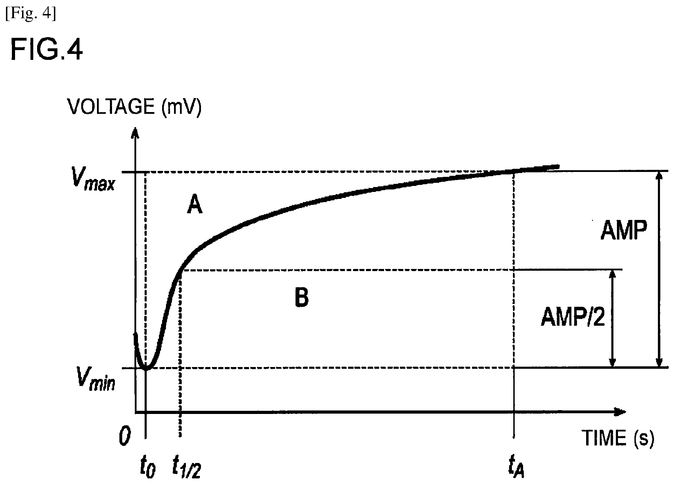

FIG. 1 is a rough diagram illustrating a measuring apparatus 1 according to the embodiment of the presently disclosed subject matter. FIG. 2 is a rough diagram illustrating a tube pump 20 of the measuring apparatus 1 according to the embodiment. FIG. 3 is a rough diagram illustrating a detection unit 50 of the measuring apparatus 1 according to the embodiment. FIG. 4 is a graph of an example syllectogram.

The measuring apparatus 1 is an apparatus for measuring a syllectogram (see Fig. 4) of a blood sample. As illustrated in FIG. 1, the measuring apparatus 1 can include a storage unit 10, the tube pump (corresponds to a term "pump") 20, a flow passage 30, a detection unit (a detector) 50, a sensor 60, and a control unit (a controller) 70.

A blood sample or a rinse liquid is stored in the storage unit 10. The storage unit 10 is a cup specifically. A blood sample or a rinse liquid acquired from a blood collection tube (not shown) is moved to the storage unit 10 by a nozzle (not shown).

The blood sample is sampled from a patient in advance and put into the blood collection tube. For example, EDTA (ethylenediaminetetraacetic acid) may be added, as an anticoagulant, to the blood sample in the blood collection tube.

As shown in FIG. 1, the tube pump 20 is disposed downstream of the flow passage 30. The tube pump 20 sucks the blood sample or rinse liquid stored in the storage unit 10. The rinse liquid is a liquid for rinsing the blood sample existing in the flow passage 30. As shown in FIG. 2, the tube pump 20 can include three rollers 21 arranged in the circumferential direction at intervals of 120°, a rotary portion 22 for rotating the three rollers 21 in the circumferential direction, and a tube 23 which is connected to the flow passage 30.

When rotating in the circumferential direction, the three rollers 21 of the tube pump 20 presses the tube 23, whereby the blood sample is sucked.

A certain shearing stress acts on the blood sample in the storage unit 10 when sucked by the tube pump 20. The blood sample is sucked when the tube pump 20 is rotated in the normal direction and is returned when the tube pump 20 is rotated in the reverse direction. Immediately before a syllectogram measurement, a temperature adjustment is performed and the blood sample is moved forward and backward in the traveling direction of the flow passage 30 by rotating the tube pump 20 in the normal direction and the reverse direction by the control unit 70.

As shown in FIG. 1, the flow passage 30 is disposed continuously downstream of the storage unit 10. For example, the flow passage 30 is a transparent glass tube or a resin tube. A blood sample sucked by the tube pump 20 flows through the flow passage 30.

The detection unit 50 measures a syllectogram as shown in FIG. 4 by detecting an intensity of transmission light setting, as a start point, a time point before a stop of a flow of a blood sample flowing through the flow passage 30. The start point of detection by the detection unit 50 is not limited to a time point before a stop of a flow of a blood sample through the flow passage 30 and may be set at a desired time point such as immediately after the stop.

As illustrated in FIG. 3, the detection unit 50 can include irradiation units (light irradiators) 51 for irradiating the flow passage 30 with light, light detecting units (light detectors) 52 for detecting light that has been emitted from the irradiation units 51 and transmitted through the flow passage 30, an optical cell 53 through which a blood sample flows, a holding unit 54 for holding the optical cell 53, and a temperature adjusting unit (not shown) for adjusting the temperature of the optical cell 53. In this embodiment, a flow passage in the optical cell 53 is a part of the flow passage 30. The flow passage 30 and the optical cell 53 may be provided separately. The optical cell 53 may be a transparent glass tube and the other part of the flow passage 30 may be a resin tube.

Two irradiation units 51 and two light detecting units 52 are provided. For example, the irradiation units 51 are near infrared light generators. The light detecting units 52 detect intensities of light beams transmitted through a blood sample. For example, the light detecting units 52 are photodiodes. The temperature adjusting unit is a heater and is disposed on, for example, the outer circumferential surface of the holding unit 54. The temperature adjusting unit makes it possible to keep the temperature of the blood sample in the optical cell 53 constant and thereby measure a syllectogram in an appropriate manner.

Since the two irradiation units 51 and the two light detecting units 52 are provided, a syllectogram of the blood sample can be measured at two locations. Thus, by comparing two measured syllectograms, if, for example, one syllectogram is different from a predetermined graph, it is possible to judge that it has not been measured properly due to inclusion of air bubbles or a contamination and to employ the other syllectogram.

As shown in FIG. 4, the horizontal axis and the vertical axis of a syllectogram represent time and the transmission light intensity, respectively. In the example syllectogram shown in FIG. 4, the output voltage of a photodiode used as a light detecting unit 52 is employed as representing the transmission light intensity.

In a syllectogram, the transmission light intensity takes a minimum value Vₘᵢₙ at a time (t = t₀) a little after a stop (t = 0) of a flow of a blood sample flowing through the flow passage 30. The transmission light intensity takes the minimum value Vₘᵢₙ because irradiation light is scattered and absorbed by individual erythrocytes dissociated and dispersed in the flow passage 30. On the other hand, at the instant (t = 0) when the flow is stopped, erythrocytes are deformed and oriented in the flow direction by a shearing stress produced by the flow and hence the transmission light intensity is a little higher than Vₘᵢₙ.

The transmission light intensity increases after taking the minimum value Vₘᵢₙ at time t₀. This is because erythrocytes start aggregating upon the stop of the flow and irradiation light passes between aggregated (i.e., size-increased) erythrocytes. Erythrocytes aggregate because blood proteins such as fibrinogen that are charged positively and have increased in number by inflammation obstruct repulsion between erythrocytes that are charged negatively.

A parameter relating to aggregation of erythrocytes (hereinafter referred to as an "aggregation parameter") is calculated on the basis of the syllectogram. The aggregation parameter may be any of a variety of parameters that are correlated with aggregation of erythrocytes.

To calculate an aggregation parameter, time t_{A} is set that is a predetermined time after time t₀. In a syllectogram, the predetermined time can be set to a desired time that is required by the increase rate of the transmission light intensity to decrease to some extent to saturate the transmission light intensity. A transmission light intensity at time t_{A} is set as a maximum value Vₘₐₓ in calculating an aggregation parameter. The aggregation parameter may be a parameter AI that is calculated in the following manner on the basis of the syllectogram. The parameter AI is calculated as a ratio of the area of a region B that is a part, located under the syllectogram curve, of a rectangular region S that has a time interval t_{A} - t₀ as one side and the difference AMP between the maximum value Vₘₐₓ and the minimum value Vₘᵢₙ as another side in the syllectogram to the region S. That is, the parameter AI is calculated as a ratio of the area of the region B to the sum of the areas of regions A and B (i.e., B/(A + B)). The region A is a region that is a part, located over the syllectogram curve, of the region S, instead of AI. The aggregation parameter may be the area of the region B, the area of the region A, AMP, or time t_{1/2}. Time t_{1/2} is a time when the transmission light intensity has increased by AMP/2 from the minimum value Vₘᵢₙ at time t₀.

As illustrated in FIG. 1, the sensor 60 is disposed between the storage unit 10 and the detection unit 50. The sensor 60 is a sensor for detecting presence/absence of an air layer or liquid in a portion, continuous with the storage unit 10, of the flow passage 30. That is, the sensor 60 detects switching from liquid to gas in the flow passage 30.

According to the invention, an impedance sensor is used as the sensor 60. The sensor 60 connects a storage unit 10-side end portion of the flow passage 30 to a portion, connected to the detection unit 50, of the flow passage 30. The sensor 60 detects a current flowing through it. If a current comes not to flow because of inflow of an air layer, the sensor 60 judges that an air layer has flown in.

The control unit 70 controls, for example, driving of the individual components. Being a CPU or the like, the control unit 70 controls the above-described various constituent elements and performs various kinds of computation etc. according to programs.

Next, a description will be made of a measuring method using the above-described measuring apparatus 1.

First, the control unit 70 supplies a rinse liquid to the storage unit 10.

Then the control unit 70 fills up the flow passage 30 with the rinse liquid by driving the tube pump 20 in the normal direction. When an air layer flows into the flow passage 30 after all of the rinse liquid that was stored in the storage unit 10 has flown into the flow passage 30, the control unit 70 detects the inflow of the air layer by means of the sensor 60.

If the sensor 60 does not detect the rinse liquid or an air layer while the tube pump 20 continues to suck the rinse liquid, the control unit 70 judges that the flow passage 30 is clogged or no rinse liquid remains.

When the sensor 60 detects inflow of an air layer, the control unit 70 stops the tube pump 20.

Then the control unit 70 acquires a blood sample from a blood collection tube by the nozzle and supplies the blood sample to the storage unit 10.

Subsequently, the control unit 70 supplies the blood sample to the detection unit 50 by driving the tube pump 20 in the normal direction. As a result, an air layer is formed between the rinse liquid and the blood sample that fill up the passage between the tube pump 20 and the sensor 60. If, for example, no air layer were formed between the rinse liquid and the blood sample and they were in direct contact with each other, the blood sample could be drawn being mixed with the rinse liquid and thereby diluted to disable an accurate syllectogram measurement. On the other hand, if a large amount of air layer were formed between the storage unit 10 and the tube pump 20, the air layer would produce high viscoelasticity and thereby lower the blood suction and stirring rates, also disabling an accurate syllectogram measurement. From these points of view, it is preferable that an air layer be contained by about 2 to 10 µl.

In contrast, according to the measuring method of the embodiment, since a small amount of air layer is formed between the rinse liquid and the blood sample, the blood sample is not mixed with the rinse liquid and the blood stirring rate is not lowered, whereby a syllectogram can be measured accurately.

Then the control unit 70 moves the blood sample forward and backward by rotating the tube pump 20 in the normal direction and the reverse direction so as to prevent a start of aggregation of the blood sample.

Then the control unit 70 stops the driving of the tube pump 20 at a timing of measurement of a syllectogram. A blood flow of the blood sample can be stopped quickly by using the tube pump 20.

Subsequently, a syllectogram of the blood sample is measured by the detection unit 50. Since the detection unit 50 has the two irradiation units 51 and the two light detecting units 52, a syllectogram can be measured at two positions. Thus, if it is found by comparing two measured syllectograms that, for example, one syllectogram is different from a predetermined graph, it is possible to judge that the one syllectogram was not measured properly due to inclusion of air bubbles or a contamination and to employ the other syllectogram. Alternatively, if one syllectogram is different from the predetermined graph, an instruction to carry out a measurement again may be issued to the user.

After the syllectogram measurement, the control unit 70 supplies a rinse liquid to the storage unit 10 again. The control unit 70 rinses the flow passage 30 by causing the tube pump 20 to suck the rinse liquid supplied. Since the sensor 60 is provided, when the step of rinsing the flow passage 30 by supplying a rinse liquid to the storage unit 10 is performed repeatedly, it is possible to detect that all of the rinse liquid in the storage unit 10 has been sucked and an air layer has flown in. It is therefore possible to supply a new rinse liquid to the storage unit 10 after a rinse liquid has been removed completely from the storage unit 10. This makes it possible to prevent, properly, an event that part of a rinse liquid spills out of the storage unit 10.

As described above, the measuring apparatus 1 according to the embodiment is a measuring apparatus for measuring aggregation of erythrocytes of a blood sample. The measuring apparatus 1 can include the storage unit 10 in which the blood sample is stored, the tube pump 20 which sucks the blood sample from the storage unit 10, the flow passage 30 which communicates with the storage unit 10 and through which the blood sample or a rinse liquid flows when sucked by the tube pump 20, the detection unit 50 which can include the irradiation unit 51 that irradiates the flow passage 30 with light and the light detecting unit 52 that detects light that has been emitted from the irradiation unit 51 and has transmitted through the flow passage 30, the sensor 60 which is disposed between the storage unit 10 and the detection unit 50 and detects switching from a liquid to a gas in the flow passage 30, and the control unit 70 which controls a stop of driving of the tube pump 20. The thus-configured measuring apparatus 1 can detect, by the sensor 60, that an air layer has entered the flow passage after a rinse liquid. Thus, an air layer of such an amount as not to obstruct a blood flow control can be formed between blood and the rinse liquid by sucking the blood after a small amount of air layer. As is understood from the above, blood can be moved accurately and contact between a rinse liquid and blood can be prevented.

The control unit 70 stops the tube pump 20 after detecting that an air layer has entered the flow passage 30 by the sensor 60 after the rinse liquid stored so far in the storage unit 10 moved to the flow passage 30. In the thus-configured measuring apparatus 1, an air layer can be formed reliably between blood and a rinse liquid.

The control unit judges that the flow passage 30 is clogged or no rinse liquid remains if the sensor 60 does not detect the rinse liquid or an air layer while the tube pump 20 is sucking the rinse liquid. The thus-configured measuring apparatus 1 can detect clogging of the flow passage 30 or absence of a rinse liquid reliably.

Two irradiation units 51 and two light detecting unit 52 are provided, and the control unit 70 compares syllectogram waveforms acquired by the two respective light detecting units 52. According to the thus-configured measuring apparatus 1, if it is found by comparing two measured syllectograms that, for example, one syllectogram is different from a predetermined graph, it is possible to judge that the one syllectogram was not measured properly due to inclusion of air bubbles or a contamination and to present information that urges the user to carry out another measurement or employ the other syllectogram.

Furthermore, as described above, the measuring method according to the embodiment is a measuring method of the measuring apparatus 1 for measuring a syllectogram, the measuring apparatus 1 being capable of including the storage unit 10 in which a blood sample is stored, the tube pump 20 which sucks the blood sample from the storage unit 10, the flow passage 30 which communicates with the storage unit 10 and through which the blood sample flows when sucked by the tube pump 20, the detection unit 50 which can include the irradiation unit 51 that irradiates the flow passage 30 with light and the light detecting unit 52 that detects light that has been emitted from the irradiation unit 51 and has transmitted through the flow passage 30, the sensor 60 which is disposed between the storage unit 10 and the detection unit 50 and detects switching from a liquid to a gas in the flow passage 30, and the control unit 70 which controls a stop of driving of the tube pump 20. The measuring method stops the tube pump 20 after detecting that an air layer has entered the flow passage 30 by the sensor 60 after the rinse liquid stored so far in the storage unit 10 moved to the flow passage 30. According to this measuring method, the sensor 60 can detect that an air layer has entered after a rinse liquid. Thus, an air layer can be formed between blood and the rinse liquid by sucking the blood after the air layer. As is understood from the above, blood can be moved accurately and contact between a rinse liquid and blood can be prevented.

### <Modification 1>

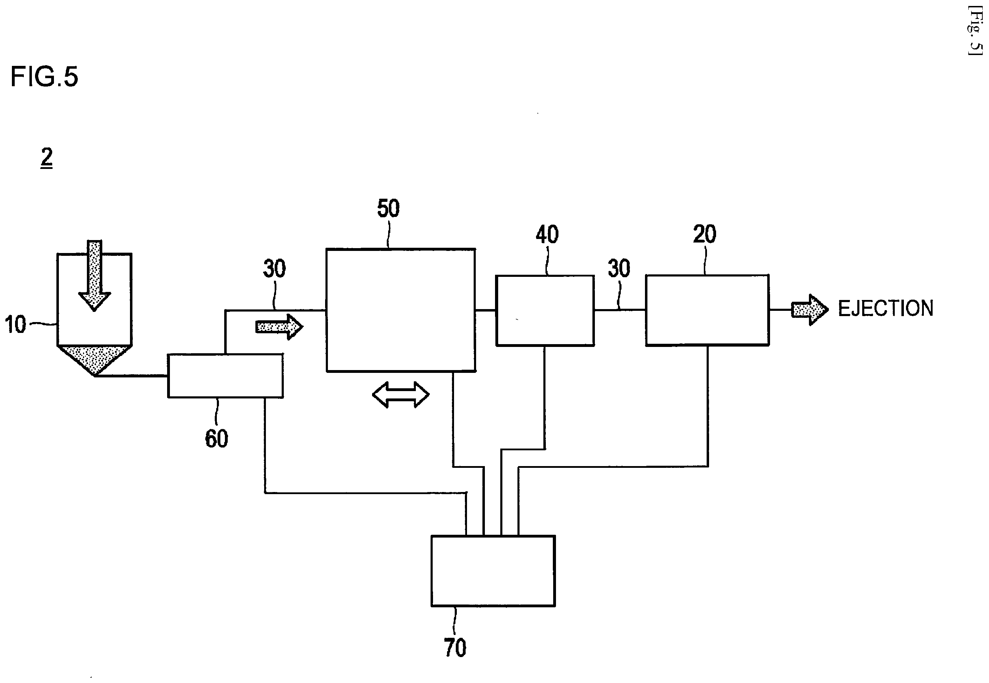

Next, the configuration of a measuring apparatus 2 according to Modification 1 will be described with reference to FIG. 5. FIG. 5 is a rough diagram illustrating the measuring apparatus 2 according to Modification 1. The measuring apparatus 2 according to Modification 1 is different from the measuring apparatus 1 according to the above-described embodiment in being provided with a valve 40. Constituent elements having the same ones in the measuring apparatus 1 according to the above-described embodiment will be given the same symbols as the latter and will not be described.

As shown in FIG. 5, the measuring apparatus 2 according to Modification 1 can include a storage unit 10, a tube pump 20, a flow passage 30, the valve 40, a detection unit 50, a sensor 60, and a control unit (a controller) 70. Since the storage unit 10, the tube pump 20, the flow passage 30, the detection unit (a detector) 50, the sensor 60, and the control unit 70 are configured in the same manners as those of the measuring apparatus 1 according to the above-described embodiment and hence will not be described.

As shown in FIG. 5, the valve 40 is disposed between the detection unit 50 and the tube pump 20. By opening the valve 40, it is possible to cause a blood sample or a rinse liquid for rinsing a blood sample remaining in the flow passage 30 to flow through the flow passage 30 by opening the flow passage 30. On the other hand, by closing the valve 40, flow of a blood sample or a rinse liquid through the flow passage 30 can be stopped by shutting off the flow passage 30. The valve 40 is opened and closed by the control unit 70.

Next, a measuring method of the measuring apparatus 2 according to the above-described Modification 1 will be described.

First, the control unit 70 supplies a rinse liquid to the storage unit 10.

Then the control unit 70 fills up the flow passage 30 with the rinse liquid by driving the tube pump 20 in the normal direction in a state that the valve 40 is open. The sensor 60 detects inflow of an air layer that occurs after inflow of the entire rinse liquid stored so far in the storage unit 10 into the flow passage 30.

The control unit 70 closes the valve 40 when the sensor 60 has detected the inflow of the air layer. The control unit 70 stops the tube pump 20 immediately after closing the valve 40.

Then the control unit 70 acquires a blood sample from a blood collection tube by the nozzle and supplies it to the storage unit 10.

Subsequently, the control unit 70 opens the valve 40 and supplies the blood sample to the detection unit 50 by driving the tube pump 20 in the normal direction. As a result, an air layer is formed between the rinse liquid and the blood sample that fill up the passage between the tube pump 20 and the sensor 60.

Then the control unit 70 moves the blood sample forward and backward with respect to the flow passage 30 by rotating the tube pump 20 in the normal direction and the reverse direction so that aggregation of the blood sample does not start.

Then the control unit 70 closes the valve 40 at a timing of measurement of a syllectogram and stops the driving of the tube pump 20 immediately thereafter. At this time, the blood flow of the blood sample can be stopped more quickly than in the measuring apparatus 1 according to the above-described embodiment.

The steps to follow will not be described because they are the same as in the measuring apparatus 1 according to the above-described embodiment.

### <Modification 2>

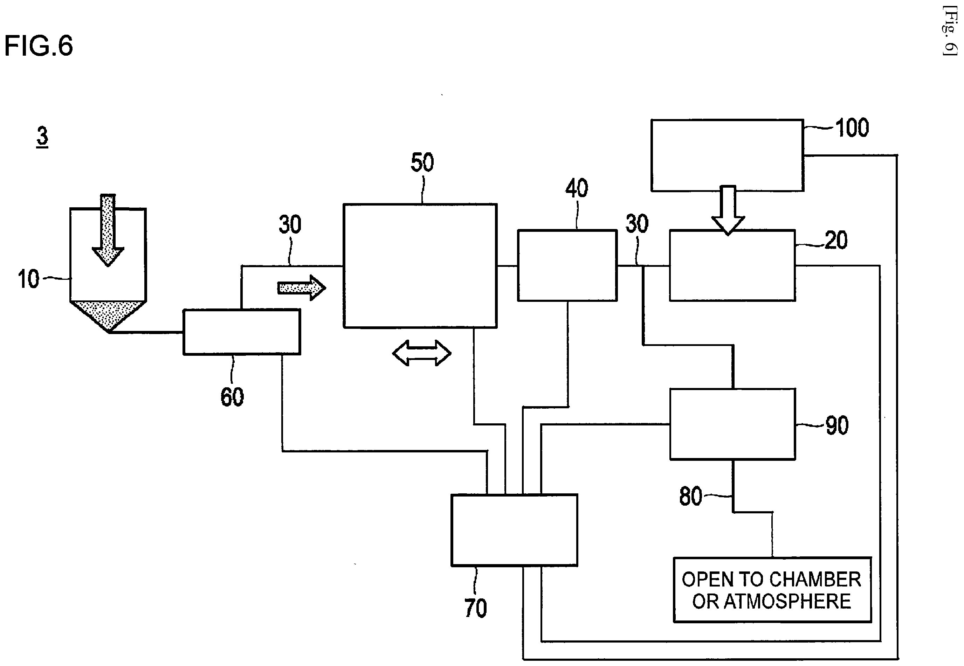

Next, the configuration of a measuring apparatus 3 according to Modification 2 will be described with reference to FIG. 6. FIG. 6 is a rough diagram illustrating the measuring apparatus 3 according to Modification 2. The measuring apparatus 3 according to Modification 2 is different from the measuring apparatus 2 according to Modification 1 in being provided with an initialization flow passage 80 etc. Constituent elements having the same ones in the measuring apparatus 1 according to the above-described embodiment or the above-described measuring apparatus 2 according to Modification 1 will be given the same symbols as the latter and will not be described.

As shown in FIG. 6, the measuring apparatus 3 according to Modification 2 can include a storage unit 10, a tube pump 20, a flow passage 30, a valve 40, a detection unit (detector) 50, a sensor 60, a control unit (a controller) 70, the initialization flow passage 80, an on-off valve 90, and a detection sensor 100. Since the storage unit 10, the tube pump 20, the flow passage 30, the valve 40, the detection unit 50, the sensor 60, and the control unit 70 are configured in the same manners as those of the measuring apparatus 2 according to the above-described Modification 1 and hence will not be described.

As described in the above embodiment, a blood sample is sucked by the detection unit 50 as the three rollers 21 of the tube pump 20 are rotated in the circumferential direction and thereby press the tube 23. As a result, depending on portions crushed by the rollers 21, there may occur a problem that the flow rate lowers temporarily, as a result of which blood cannot be suction-controlled so as to reach a correct position or the shearing pressure cannot be controlled to a predetermined value for dissociation of erythrocytes that is important for syllectogram measurement. Thus, in stirring a blood sample by moving it forward and backward by rotating the tube pump 20 in the normal direction and the reverse direction, it is necessary to set (initialize) the flowrate-reducing crushing positions of the rollers 21 at predetermined initial positions. Furthermore, to suck blood to a predetermined position in the detection unit 50 and to initialize the tube pump 20, it is necessary to drive the tube pump 20 independently of blood suction. It is therefore necessary to shut off the flow passage at a position between the storage unit 10 and the tube pump 20 and to switch to a different flow passage.

The configuration of the measuring apparatus 3 according to Modification 2 will be described below. The initialization flow passage 80 branches off at a position between the valve 40 and the tube pump 20. The initialization flow passage 80 is open to a large chamber or the atmosphere.

The on-off valve 90 is disposed in the initialization flow passage 80. A blood sample or a rinse liquid can be caused to flow into the initialization flow passage 80 by opening the initialization flow passage 80 by closing the valve 40 and opening the on-off valve 90. On the other hand, flow of a blood sample or a rinse liquid through the initialization flow passage 80 can be stopped by shutting off the initialization flow passage 80 by opening the valve 40 and closing the on-off valve 90. The opening/ closing of the on-off valve 90 is controlled by the control unit 70.

The detection sensor 100 detects whether the rollers 21 of the tube pump 20 are located at predetermined positions. There are no particular limitations on the type of the detection sensor 100 except that it should be a sensor capable of detecting whether the rollers 21 are located at the predetermined positions. For example, the detection sensor 100 may be an encoder, a flow sensor, a magnetic sensor, or a distance sensor.

Where the detection sensor 100 is an encoder, it outputs a pulse signal according to rotation of the tube pump 20, that is, every rotation angle 120° of the tube pump 20, to the control unit 70. Positions of the rollers 21 can thus be checked.

Where the detection sensor 100 is a flow sensor, positions of the rollers 21 can be checked on the basis of a flow rate of a rinse liquid flowing in the vicinity of the tube pump 20.

Next, a measuring method of the measuring apparatus 3 according to Modification 2 will be described. The measuring method of the measuring apparatus 3 according to Modification 2 is the same as that of the measuring apparatus 2 according to Modification 1 to the step of forming an air layer between a rinse liquid and a blood sample.

After an air layer is formed between a rinse liquid and a blood sample, the control unit 70 closes the valve 40 and opens the on-off valve 90. Then the control unit 70 initializes the positions of the rollers 21 on the basis of information obtained by the detection sensor 100 by driving the tube pump 20. By initializing the positions of the rollers 21 in this manner, the blood sample can be moved forward and backward properly by rotating the tube pump 20 in the normal direction and the reverse direction.

Subsequently, the control unit 70 opens the valve 40 and closes the on-off valve 90. Then the control unit 70 starts suction of the blood to the detection unit 70 and forward and backward movement of the blood. The steps to follow are the same as in the measuring method of the measuring apparatus 2 according to Modification 1.

The presently disclosed subject matter is not limited to the above-described embodiment and Modifications and various modifications are possible within the confines of the claims.

For example, a configuration is possible in which the valve 40 which is employed in the above-described Modifications is made a three-way valve that is connected to the detection unit 50, the tube pump 20, and the initialization flow passage 80. This configuration can simplify the measuring apparatus.

Whereas the two irradiation units 51 and the two light detecting units 52 are provided in the above-described embodiment, three irradiation units 51 and three two light detecting units 52 may be provided. As a further alternative, one irradiation unit 51 and one light detecting unit 52 may be provided.

In the above-described embodiment, each of the light detecting units 52 detects an intensity of transmission light transmitted from a blood sample. Alternatively, each light detecting unit may detect an intensity of reflection light reflected from a blood sample.

Whereas in the above-described embodiment the tube pump is employed as an example pump, another type of pump may be employed.

Whereas in the above-described embodiment the nozzle is provided separately from the storage unit 10, the nozzle may be integrated with the storage unit.

This application claims priority to Japanese Patent Application No. 2020-014053 filed on January 30, 2020.

## Claims

1. A measuring apparatus (1) for measuring aggregation of erythrocytes of a blood sample, comprising:
a storage unit (10) which is configured to selectively store the blood sample and a rinse liquid;
a pump (20) which is configured to suck the blood sample or the rinse liquid from the storage unit (10);
a flow passage (30) which is configured to communicate with the storage unit (10) and through which the blood sample and the rinse liquid flows when sucked by the pump (20);
a detection unit (50) including an irradiation unit (51) which is configured to irradiate the flow passage (30) with light and a light detecting unit (52) which is configured to detect light that has been emitted from the irradiation unit (51) and has been transmitted through or been reflected from the flow passage (30);
an impedance sensor (60) which is disposed between the storage unit (10) and the detection unit (50) and is configured to detect switching from the rinse liquid or the blood sample to a gas in the flow passage (30); and
a control unit (70) which is configured to control a stop of driving of the pump (20) in response to a detection by the impedance sensor (60) detecting switching from the rinse liquid or the blood sample to the gas;
wherein the end of the flow passage (30) on the storage unit (10) side and the portion of the flow passage (30) connected to the detection unit (50) are connected to each other via the impedance sensor (60), and while detecting a current flowing, if a current comes not to flow because of inflow of an air layer, the impedance sensor (60) is configured to judge that an air layer has flown in.

2. The measuring apparatus (1) according to claim 1, wherein the control unit (70) is configured to stop the pump (20) after detecting that an air layer has entered the flow (30) passage by the sensor (60) after the rinse liquid stored in the storage unit (10) moved to the flow passage (30).

3. The measuring apparatus (1) according to claim 2, wherein the control unit (70) is configured to judge that the flow passage (30) is clogged or no rinse liquid remains if the sensor (60) does not detect the rinse liquid or does detect an air layer while the pump (20) continues to suck the rinse liquid.

4. The measuring apparatus (1) according to any one of claims 1 to 3, wherein:
the pump (20) is a tube pump; and
the measuring apparatus (1) further comprises a valve (40) which is configured, in a closed state, to stop a flow of the blood sample or the rinse liquid through the flow passage (30).

5. The measuring apparatus (1) according to claim 4, wherein the control unit (70) is configured to stop the pump (20) after switching the valve (40) so as to shut off the flow passage (30).

6. The measuring apparatus (1) according to any one of claims 1 to 5, wherein:
the irradiation unit (51) and the light detecting unit (52) are provided in plurality; and
the control unit (70) is configured to compare syllectogram waveforms acquired by the plural light detecting units (52).

7. The measuring apparatus (1) according to any one of claims 4 to 6, wherein:
the pump (20) is a tube pump; and
the measuring apparatus (1) further comprises:
an initialization flow passage (80) which is provided so as to branch off at a position between the valve (40) and the tube pump (20);
an on-off valve (90) which is disposed in the initialization flow passage (80) and is configured to switch between opening and shutting-off of the initialization flow passage (80); and
a detection sensor (100) which is configured to detect whether rollers (21) of the tube pump (20) are located at predetermined positions.

8. The measuring apparatus (1) according to claim 7, wherein the detection sensor (100) is at least one of an encoder and a flow sensor.

9. The measuring apparatus (1) according to claim 7 or 8, wherein the valve (40) is a three-way valve that is connected to the detection unit (50), the tube pump (20), and the initialization flow passage (80).

10. The measuring apparatus (1) according to any one of claims 1 to 9, further comprising a temperature adjusting unit which is configured to adjust the temperature of the blood sample in the flow passage (30) to a predetermined temperature.

11. A measuring method of a measuring apparatus (1) for measuring aggregation of erythrocytes of a blood sample, the measuring apparatus (1) comprising:
a storage unit (10) in which the blood sample and a rinse liquid are selectively stored;
a pump (20) which sucks the blood sample or the rinse liquid from the storage unit (10);
a flow passage (30) which communicates with the storage unit (10) and through which the blood sample and the rinse liquid flows when sucked by the pump (20);
a detection unit (50) including an irradiation unit (51) which irradiates the flow passage (30) with light and a light detecting unit (52) which detects light that has been emitted from the irradiation unit (50) and has transmitted through or been reflected from the flow passage (30);
an impedance sensor (60) which is disposed between the storage unit (10) and the detection unit (50) and detects switching from the rinse liquid or the blood sample to a gas in the flow passage (30), and
a control unit (70) which controls a stop of driving of the pump (20) in response to the impedance sensor (60) detecting switching from the rinse liquid or the blood sample to the gas,
wherein the end of the flow passage (30) on the storage unit (10) side and the portion of the flow passage (30) connected to the detection unit (50) are connected to each other via the impedance sensor (60), and while detecting a current flowing, if a current comes not to flow because of inflow of an air layer, the impedance sensor (60) is configured to judge that an air layer has flown in,
wherein:
the measuring method includes stopping the pump (20) after detecting switching from the rinse liquid or the blood sample to the gas in the flow passage (30) by the impedance sensor (60) after the rinse liquid or the blood sample stored in the storage unit (10) moved to the flow passage (30).

12. The measuring apparatus (1) according to claim 1, wherein the control unit (70) is configured to acquire the blood from a blood collection tube by a nozzle and to supply the blood to the storage unit (10) in response to stopping of driving of the pump (20) in response to the impedance sensor (60) detecting switching from the rinse liquid to the gas.

13. The measuring method according to claim 11, wherein the control unit (70) acquires the blood from a blood collection tube by a nozzle and supplies the blood to storage unit (10) in response to stopping of driving of the pump (20) in response to the impedance sensor (60) detecting switching from the rinse liquid to the gas.

## Patentansprüche

1. Messvorrichtung (1) zum Messen von Aggregation von Erythrozyten einer Blutprobe, umfassend:
eine Speicherungs-Einheit (10), die so ausgeführt ist, dass sie selektiv die Blutprobe und eine Spülflüssigkeit speichert;
eine Pumpe (20), die so ausgeführt ist, dass sie die Blutprobe oder die Spülflüssigkeit aus der Speicherungs-Einheit (10) ansaugt;
einen Strömungskanal (30), der so ausgeführt ist, dass er mit der Speicherungs-Einheit (10) in Verbindung steht, und durch den die Blutprobe sowie die Spülflüssigkeit strömen, wenn sie durch die Pumpe (20) angesaugt werden;
eine Erfassungs-Einheit (50), die eine Bestrahlungs-Einheit (51), die zum Bestrahlen des Strömungskanals (30) mit Licht ausgeführt ist, sowie eine Licht erfassende Einheit (52) einschließt, die so ausgeführt ist, dass sie Licht erfasst, das von der Bestrahlungs-Einheit (51) emittiert worden und das durch den Strömungskanal (30) durchgelassen oder von diesem reflektiert worden ist;
einen Impedanz-Sensor (60), der zwischen der Speicherungs-Einheit (10) und der Erfassungs-Einheit (50) angeordnet und so ausgeführt ist, dass er Wechsel von der Spülflüssigkeit oder der Blutprobe zu einem Gas in dem Strömungskanal (30) erfasst; sowie
eine Steuerungs-Einheit (70), die so ausgeführt ist, dass sie eine Unterbrechung von Antrieb der Pumpe (20) in Reaktion auf eine Erfassung durch den Impedanz-Sensor (60) steuert, mit der Wechsel von der Spülflüssigkeit oder der Blutprobe zu dem Gas erfasst wird;
wobei das Ende des Strömungskanals (30) an der Seite der Speicherungs-Einheit (10) und der mit der Erfassungs-Einheit (50) verbundene Abschnitt des Strömungskanals (30) miteinander über den Impedanz-Sensor verbunden sind, und der Impedanz-Sensor (60) so ausgeführt ist, dass er, während Fließen eines Stroms erfasst wird, wenn ein Strom aufgrund von Einströmen einer Luftschicht nicht zum Strömen kommt, feststellt, dass eine Luftschicht eingeströmt ist.

2. Messvorrichtung (1) nach Anspruch 1, wobei die Steuerungs-Einheit (80) so ausgeführt ist, dass sie die Pumpe (20) anhält, nachdem sie mit dem Sensor (60) erfasst hat,
dass eine Luftschicht in den Strömungskanal (30) eingetreten ist, nachdem die in der Speicherungs-Einheit (10) gespeicherte Spülflüssigkeit zu dem Strömungskanal (30) gelangt ist.

3. Messvorrichtung (1) nach Anspruch 2, wobei die Steuerungs-Einheit (70) so ausgeführt ist, dass sie feststellt, dass der Strömungskanal (30) verstopft ist oder keine Spülflüssigkeit zurückbleibt, wenn der Sensor (60) die Spülflüssigkeit nicht erfasst oder eine Luftschicht erfasst, während die Pumpe (20) die Spülflüssigkeit weiterhin ansaugt.

4. Messvorichtung (1) nach einem der Ansprüche 1 bis 3, wobei:
die Pumpe (20) eine Schlauchpumpe ist; und
die Messvorrichtung (1) des Weiteren ein Ventil (40) umfasst, das so ausgeführt ist, dass es in einem geschlossenen Zustand einen Strom der Blutprobe oder der Spülflüssigkeit durch den Strömungskanal (30) unterbricht.

5. Messvorrichtung (1) nach Anspruch 4, wobei die Steuerungs-Einheit (70) so ausgeführt ist, dass sie die Pumpe (20) nach Umschalten des Ventils (40) zum Sperren des Strömungskanals (30) anhält.

6. Messvorichtung (1) nach einem der Ansprüche 1 bis 5, wobei:
die Bestrahlungs-Einheit (51) und die Licht erfassende Einheit (52) mehrfach vorhanden sind; und
die Steuerungs-Einheit (70) so ausgeführt ist, dass sie durch die mehreren Licht erfassenden Einheiten (52) erfasste Syllectogramm-Wellenformen vergleicht.

7. Messvorichtung (1) nach einem der Ansprüche 4 bis 6, wobei:
die Pumpe (20) eine Schlauchpumpe ist; und
die Messvorichtung (1) des Weiteren umfasst:
einen Einleitungs-Strömungskanal (80), der so eingerichtet ist, dass er an einer Position zwischen dem Ventil (40) und der Schlauchpumpe (20) abzweigt;
ein Schaltventil (90), das in dem Einleitungs-Strömungskanal (80) angeordnet und so ausgeführt ist, dass es zwischen Öffnen und Sperren des Einleitungs-Strömungskanals (80) umschaltet; sowie
einen Erfassungs-Sensor (100), der so ausgeführt ist, dass er erfasst, ob Walzen (21) der Schlauchpumpe (20) sich an vorgegebenen Positionen befinden.

8. Messvorrichtung (1) nach Anspruch 7, wobei der Erfassungssensor (100) ein Codierer oder/und ein Durchflusssensor ist.

9. Messvorrichtung (1) nach Anspruch 7 oder 8, wobei das Ventil (40) ein Dreiwegeventil ist, das mit der Erfassungs-Einheit (50), der Schlauchpumpe (20) und dem Einleitungs-Strömungskanal (80) verbunden ist.

10. Messvorichtung (1) nach einem der Ansprüche 1 bis 9, die des Weiteren eine Temperaturregulier-Einheit umfasst, die zum Regulieren der Temperatur der Blutprobe in dem Strömungskanal (30) auf eine vorgegebene Temperatur ausgeführt ist

11. Messverfahren einer Messvorrichtung (1) zum Messen von Aggregation von Erythrozyten einer Blutprobe,
wobei die Messvorrichtung (1) umfasst:
eine Speicherungs-Einheit (10), in der die Blutprobe und eine Spülflüssigkeit selektiv gespeichert werden;
eine Pumpe (20), die die Blutprobe oder die Spülflüssigkeit aus der Speicherungs-Einheit (10) ansaugt;
einen Strömungskanal (30), der mit der Speicherungs-Einheit (10) in Verbindung steht, und durch den die Blutprobe und die Spülflüssigkeit strömen, wenn sie durch die Pumpe (20) angesaugt werden;
eine Erfassungs-Einheit (50), die eine Bestrahlungs-Einheit (51), die den Strömungskanal (30) mit Licht bestrahlt, sowie eine Licht erfassende Einheit (52) einschließt, die Licht erfasst, das von der Bestrahlungs-Einheit (51) emittiert worden ist und das durch den Strömungskanal (30) durchgelassen oder von diesem reflektiert worden ist;
einen Impedanz-Sensor (60), der zwischen der Speicherungs-Einheit (10) und der Erfassungs-Einheit (50) angeordnet ist und Wechsel von der Spülflüssigkeit oder der Blutprobe zu einem Gas in dem Strömungskanal (30) erfasst; sowie
eine Steuerungs-Einheit (70), die eine Unterbrechung von Antrieb der Pumpe (20) in Reaktion darauf erfasst, dass der Impedanz-Sensor (60) Wechsel von der Spülflüssigkeit oder der Blutprobe zu dem Gas erfasst,
wobei das Ende des Strömungskanals (30) an der Seite der Speicherungs-Einheit (10) und der mit der Erfassungs-Einheit (50) verbundene Abschnitt des Strömungskanals (30) miteinander über den Impedanz-Sensor (60) verbunden sind, und der Impedanz-Sensor (60) so ausgeführt ist, dass er, während Fließen eines Stroms erfasst wird, wenn ein Strom aufgrund von Einströmen einer Luftschicht nicht zum Strömen kommt, feststellt, dass eine Luftschicht eingeströmt ist.
wobei:
das Messverfahren einschließt, dass die Pumpe (20) nach Erfassen von Wechsel von der Spülflüssigkeit oder der Blutprobe zu dem Gas in dem Strömungskanal (30) durch den Impedanz-Sensor (60) angehalten wird, nachdem die Spülflüssigkeit oder die Blutprobe, die in der Speicherungs-Einheit (10) gespeichert ist, zu dem Strömungskanal (30) gelangt ist.

12. Messvorrichtung (1) nach Anspruch 1, wobei die Steuerungs-Einheit (70) so ausgeführt ist, dass sie das Blut über einen Blut-Sammelschlauch mittels einer Düse bezieht und das Blut in Reaktion auf Anhalten von Antrieb der Pumpe (20) der Speicherungs-Einheit (10) in Reaktion darauf zuführt, dass der Impedanz-Sensor (60) Wechsel von der Spülflüssigkeit zu dem Gas erfasst.

13. Messverfahren nach Anspruch 11, wobei die Steuerungs-Einheit (70) das Blut über einen Blut-Sammelschlauch mittels einer Düse bezieht und das Blut in Reaktion auf Anhalten von Antrieb der Pumpe (20) der Speicherungs-Einheit (10) in Reaktion darauf zuführt, dass der Impedanz-Sensor (60) Wechsel von der Spülflüssigkeit zu dem Gas erfasst.

## Revendications

1. Appareil de mesure (1) pour mesurer une agrégation d'érythrocytes d'un échantillon de sang, comprenant :
une unité de stockage (10) qui est configurée pour stocker sélectivement l'échantillon de sang et un liquide de rinçage ;
une pompe (20) qui est configurée pour aspirer l'échantillon de sang ou le liquide de rinçage depuis l'unité de stockage (10) ;
un passage d'écoulement (30) qui est configuré pour communiquer avec l'unité de stockage (10) et à travers lequel l'échantillon de sang et le liquide de rinçage circulent lorsqu'ils sont aspirés par la pompe (20) ;
une unité de détection (50) comportant une unité d'irradiation (51) qui est configurée pour irradier le passage d'écoulement (30) avec de la lumière et une unité de détection de lumière (52) qui est configurée pour détecter de la lumière qui a été émise par l'unité d'irradiation (51) et qui a été transmise à travers le passage d'écoulement (30) ou réfléchie par celui-ci ;
un capteur d'impédance (60) qui est disposé entre l'unité de stockage (10) et l'unité de détection (50) et est configuré pour détecter l'échange du liquide de rinçage ou de l'échantillon de sang par un gaz dans le passage d'écoulement (30) ; et
une unité de commande (70) qui est configurée pour commander l'arrêt de l'entraînement de la pompe (20) en réponse à une détection par le capteur d'impédance (60) de l'échange du liquide de rinçage ou de l'échantillon de sang par le gaz ;
dans lequel l'extrémité du passage d'écoulement (30) sur le côté unité de stockage (10) et la portion du passage d'écoulement (30) connectée à l'unité de détection (50) sont connectées l'une à l'autre via le capteur d'impédance (60), et tout en détectant un courant en circulation, si un courant cesse de circuler en raison de l'entrée d'une couche d'air, le capteur d'impédance (60) est configuré pour juger qu'une couche d'air est entrée.

2. Appareil de mesure (1) selon la revendication 1, dans lequel l'unité de commande (70) est configurée pour arrêter la pompe (20) après avoir détecté qu'une couche d'air s'est introduite dans le passage d'écoulement (30) par le capteur (60) après que le liquide de rinçage stocké dans l'unité de stockage (10) s'est déplacé vers le passage d'écoulement (30).

3. Appareil de mesure (1) selon la revendication 2, dans lequel l'unité de commande (70) est configurée pour juger que le passage d'écoulement (30) est obstrué ou qu'il ne reste plus de liquide de rinçage si le capteur (60) ne détecte pas le liquide de rinçage ou détecte une couche d'air tandis que la pompe (20) continue d'aspirer le liquide de rinçage.

4. Appareil de mesure (1) selon l'une quelconque des revendications 1 à 3, dans lequel :
la pompe (20) est une pompe à tube ; et
l'appareil de mesure (1) comprend en outre une valve (40) qui est configurée, dans un état fermé, pour arrêter un écoulement de l'échantillon de sang ou du liquide de rinçage à travers le passage d'écoulement (30).

5. Appareil de mesure (1) selon la revendication 4, dans lequel l'unité de commande (70) est configurée pour arrêter la pompe (20) après commutation de la valve (40) afin de fermer le passage d'écoulement (30).

6. Appareil de mesure (1) selon l'une quelconque des revendications 1 à 5, dans lequel :
l'unité d'irradiation (51) et l'unité de détection de lumière (52) sont fournies en pluralité ;
et
l'unité de commande (70) est configurée pour comparer des formes d'onde de syllectrogramme acquises par la pluralité d'unités de détection de lumière (52).

7. Appareil de mesure (1) selon l'une quelconque des revendications 4 à 6, dans lequel :
la pompe (20) est une pompe à tube ; et
l'appareil de mesure (1) comprend en outre :
un passage d'écoulement d'initialisation (80) qui est agencé de manière à se ramifier à une position entre la valve (40) et la pompe à tube (20) ;
une valve marche-arrêt (90) qui est disposée dans le passage d'écoulement d'initialisation (80) et est configurée pour commuter entre l'ouverture et la fermeture du passage d'écoulement d'initialisation (80) ; et
un capteur de détection (100) qui est configuré pour détecter si des rouleaux (21) de la pompe à tube (20) sont situés à des positions prédéterminées.

8. Appareil de mesure (1) selon la revendication 7, dans lequel le capteur de détection (100) est au moins l'un parmi un codeur et un capteur d'écoulement.

9. Appareil de mesure (1) selon la revendication 7 ou 8, dans lequel la valve (40) est une valve à trois voies qui est connectée à l'unité de détection (50), à la pompe à tube (20) et au passage d'écoulement d'initialisation (80).

10. Appareil de mesure (1) selon l'une quelconque des revendications 1 à 9, comprenant en outre une unité de réglage de température qui est configurée pour régler la température de l'échantillon de sang dans le passage d'écoulement (30) à une température prédéterminée.

11. Procédé de mesure d'un appareil de mesure (1) pour mesurer une agrégation d'érythrocytes d'un échantillon de sang, l'appareil de mesure (1) comprenant :
une unité de stockage (10) dans laquelle l'échantillon de sang et un liquide de rinçage sont stockés sélectivement ;
une pompe (20) qui aspire l'échantillon de sang ou le liquide de rinçage depuis l'unité de stockage (10) ;
un passage d'écoulement (30) qui communique avec l'unité de stockage (10) et à travers lequel l'échantillon de sang et le liquide de rinçage circulent lorsqu'ils sont aspirés par la pompe (20) ;
une unité de détection (50) comportant une unité d'irradiation (51) qui irradie le passage d'écoulement (30) avec de la lumière et une unité de détection de lumière (52) qui détecte de la lumière qui a été émise par l'unité d'irradiation (51) et qui a été transmise à travers le passage d'écoulement (30) ou réfléchie par celui-ci ;
un capteur d'impédance (60) qui est disposé entre l'unité de stockage (10) et l'unité de détection (50) et détecte l'échange du liquide de rinçage ou de l'échantillon de sang par un gaz dans le passage d'écoulement (30) ; et
une unité de commande (70) qui commande l'arrêt de l'entraînement de la pompe (20) en réponse à une détection par le capteur d'impédance (60) de l'échange du liquide de rinçage ou de l'échantillon de sang par le gaz ;
dans lequel l'extrémité du passage d'écoulement (30) sur le côté unité de stockage (10) et la portion du passage d'écoulement (30) connectée à l'unité de détection (50) sont connectées l'une à l'autre via le capteur d'impédance (60), et tout en détectant un courant en circulation, si un courant cesse de circuler en raison de l'entrée d'une couche d'air, le capteur d'impédance (60) est configuré pour juger qu'une couche d'air est entrée,
dans lequel :
le procédé de mesure comporte l'arrêt de la pompe (20) après avoir détecté l'échange du liquide de rinçage ou de l'échantillon de sang par le gaz dans le passage d'écoulement (30) par le capteur d'impédance (60) après que le liquide de rinçage ou l'échantillon de sang stocké dans l'unité de stockage (10) s'est déplacé vers le passage d'écoulement (30).

12. Appareil de mesure (1) selon la revendication 1, dans lequel l'unité de commande (70) est configurée pour acquérir le sang auprès d'un tube de prélèvement de sang par une buse et pour fournir le sang à l'unité de stockage (10) en réponse à l'arrêt de l'entraînement de la pompe (20) en réponse à la détection par le capteur d'impédance (60) de l'échange du liquide de rinçage par le gaz.

13. Procédé de mesure selon la revendication 11, dans lequel l'unité de commande (70) acquiert le sang auprès d'un tube de prélèvement de sang par une buse et fournit le sang à l'unité de stockage (10) en réponse à l'arrêt de l'entraînement de la pompe (20) en réponse à la détection par le capteur d'impédance (60) de l'échange du liquide de rinçage par le gaz.
